(19) Europäisches Patentamt
European Patent Office
Office européen des brevets

(11) Numéro de publication: **0 092 491**
**A1**

(12) # DEMANDE DE BREVET EUROPEEN

(21) Numéro de dépôt: **83420062.8**

(51) Int. Cl.³: **C 07 C 69/44**, C 07 C 67/38

(22) Date de dépôt: **06.04.83**

(30) Priorité: **07.04.82 FR 8206225**

(43) Date de publication de la demande: **26.10.83**
**Bulletin 83/43**

(84) Etats contractants désignés: **AT BE CH DE FR GB IT LI LU NL SE**

(71) Demandeur: **RHONE-POULENC CHIMIE DE BASE,**
**25, quai Paul Doumer, F-92408 Courbevoie (FR)**

(72) Inventeur: **Jenck, Jean, 11, rue Lakanal,**
**F-69100 Villeurbanne (FR)**

(74) Mandataire: **Varniere-Grange, Monique et al,**
**RHONE-POULENC RECHERCHES Centre de Recherches de Saint-Fons Service Brevets B.P. 62,**
**F-69190 Saint-Fons (FR)**

(54) **Procédé de préparation d'adipates d'alkyles.**

(57) La présente invention a pour objet un procédé de préparation d'adipates d'alkyle à partir de penténoates d'alkyle.

Selon le présent procédé, on fait réagir du monoxyde de carbone et un alcool sur un penténoate d'alkyle en présence de cobalt, d'une base azotée tertiaire et d'hydrogène, l'hydrogène représentant au moins 0,1 % (en volume) du monoxyde de carbone, et dans un hydrocarbure aromatique qui peut comporter, le cas échéant, de 1 à 3 substituants indépendamment choisis parmi les radicaux alkyles, aryles ou aralkyles renfermant au maximum 20 atomes de carbone.

EP 0 092 491 A1

PROCEDE DE PREPARATION D'ADIPATES D'ALKYLES

La présente invention a pour objet un procédé de préparation d'adipates d'alkyle à partir de pentènoates d'alkyle.

La présente invention a plus particulièrement pour objet un procédé de préparation sélective d'adipates d'alkyle par réaction du monoxyde de carbone et d'un alcool sur des pentènoates d'alkyle.

Il est bien connu, d'après le "Bulletin of the Chemical Society of Japan, vol. 46, 1973, pages 526 et 527", qu'on obtient un mélange renfermant des diesters d'alkyle et notamment, un adipate d'alkyle, en faisant réagir du monoxyde de carbone et un alcool sur un pentène-3 oate d'alkyle, sous haute pression et à température élevée, en présence de cobalt carbonyle et d'une base azotée hétérocyclique et aromatique. Cependant le développement à l'échelle industrielle d'une telle technique, dont l'intérêt de principe n'est pas contesté, est largement compromis, non seulement par la faible efficacité du système catalytique, mais aussi par la proportion notable de pentanoate d'alkyle formée, bien que la réaction soit effectuée en l'absence d'hydrogène.

Il est par ailleurs bien connu des spécialistes de ce domaine que la présence de faibles quantités d'hydrogène dans le milieu réactionnel tend à augmenter l'efficacité des catalyseurs à base de cobalt, dans les procédés de synthèse d'esters par réaction d'un alcool et du monoxyde de carbone sur un composé oléfinique.

La Demanderesse a néanmoins constaté que dans le procédé en cause cet effet favorable, lié à la présence de faibles quantités d'hydrogène, est accompagné par une influence négative sur la sélectivité en adipates d'alkyle, produits spécifiquement visés.

En effet on observe que la présence d'hydrogène non seulement tend à augmenter la proportion de produits d'hydrogénation dans le mélange réactionnel, mais aussi elle est susceptible de diminuer la proportion d'adipate parmi les diesters formés.

Cet effet négatif grève lourdement l'économie du procédé dans la mesure où la valorisation des diesters branchés et des pentanoates d'alkyle est aléatoire, voire inexistante. En d'autres termes la formation de ces produits, qui sont détruits, en pratique, correspond à

une perte intolérable de matière première. Par ailleurs de l'hydrogène peut être formé in-situ à partir des traces d'eau susceptibles d'être contenues par des réactifs de qualité technique, selon la réaction bien connue :

$$H_2O + CO \longrightarrow CO_2 + H_2$$

Il serait souhaitable, pour des raisons économiques évidentes, de pouvoir utiliser du monoxyde de carbone de qualité technique renfermant de l'hydrogène, sans que cela se produise au détriment de la sélectivité en adipates d'alkyle, diesters visés. Il serait également souhaitable, pour les mêmes raisons, de pouvoir utiliser des réactifs renfermant des traces d'eau, sans que cela conduise à une perte de matière première.

Il a maintenant été trouvé de manière tout à fait surprenante qu'il est possible de préparer sélectivement des adipates d'alkyle par réaction d'un alcool et du monoxyde de carbone sur des pentènoates d'alkyle en présence d'un catalyseur métallique choisi dans le groupe constitué par le cobalt et ses composés, d'une base azotée tertiaire et d'hydrogène, l'hydrogène représentant au moins 0,1 % en volume du monoxyde de carbone, à condition de conduire la réaction dans un hydrocarbure aromatique qui peut comporter, le cas échéant, de 1 à 3 substituants indépendamment choisis parmi les radicaux alkyles, aryles et aralkyles renfermant au maximum 20 atomes de carbone.

Selon la présente invention on fait donc réagir un alcool et du monoxyde de carbone sur un pentènoate d'alkyle. Les pentènoates d'alkyle peuvent être obtenus par réaction d'un alcool et du monoxyde de carbone sur le butadiène, de manière connue en soi. Les pentène-3 oates d'alkyle sont obtenus comme produits principaux. On peut également utiliser, dans le cadre du présent procédé, des pentène-2 oates d'alkyle, obtenus, par exemple par isomérisation des pentènes-3 oates correspondants, ces esters-$\alpha$,$\beta$ insaturés se révèlant plus réactifs.

Pour mettre en oeuvre le présent procédé, il est également fait appel à un alcool. Cette autre matière première peut être représentée par la formule R'OH dans laquelle R' est soit un radical alkyle comportant au maximum 12 atomes de carbone éventuellement substitué par un ou deux groupes hydroxyles, soit un radical cycloalkyle ayant de 5 à 7 atomes de

carbone, ou bien un radical aralkyle ayant de 7 à 12 atomes de carbone ou un radical phényle.

A titre d'exemples d'alcools utilisables dans le cadre du présent procédé on peut citer le méthanol, l'éthanol, l'iosopropanol, le n-propanol, le tertiobutanol, le n-hexanol, le cyclohexanol, l'éthyl-2 hexanol-1, le dodécanol-1, l'éthylène glycol, l'hexanediol-1,6, l'alcool benzylique, l'alcool phényléthylique et le phénol.

On utilise de préférence un alcanol ayant au maximum 4 atomes de carbone ; le méthanol et l'éthanol conviennent bien à la mise en oeuvre du présent procédé. On utilise avantageusement l'alcool correspondant au reste alkyle du pentènoate choisi comme matière de départ.

On peut engager l'alcool et le pentènoate d'alkyle en quantités stoechiométriques. Cependant on utilise de préférence un excès d'alcool, dans la proportion de 1 à 10, ou encore mieux, de 2 à 5 moles d'alcool par mole de pentènoate d'alkyle.

La réaction est conduite en présence d'un catalyseur métallique, choisi dans le groupe constitué par le cobalt et ses composés. N'importe quelle source de cobalt susceptible de réagir avec le monoxyde de carbone dans le milieu réactionnel pour donner in-situ des complexes carbonyles du cobalt peut être utilisée dans le cadre du présent procédé.

Les sources typiques de cobalt sont par exemple le cobalt métallique finement divisé, des sels inorganiques tels que le nitrate ou le carbonate de cobalt, des sels organiques en particulier des carboxylates. Peuvent également être employés les cobalt-carbonyles ou hydrocarbonyles ; le dicobaltoctacarbonyle convient bien à la mise en oeuvre du présent procédé.

Le rapport molaire entre le pentènoate d'alkyle et le cobalt est en général compris entre 10 et 1 000. Ce rapport est avantageusement fixé à une valeur comprise entre 20 et 300.

Le procédé selon la présente invention nécessite également la présence d'une base azotée tertiaire dont le $pK_a$ est compris entre 3 et 10.

La Demanderesse préconise l'utilisation d'hétérocycles azotés formés de 5 à 6 chaînons, qui peuvent comporter un ou deux substituants choisis parmi les groupes alkyles ou alcoxy ayant au maximum 4 atomes de

carbone, le groupe hydroxy et les atomes d'halogène, qui renferment éventuellement 2 ou 3 doubles liaisons, et qui peuvent, par ailleurs, le cas échéant, être soudés à un noyau benzénique, pourvu que les maillons adjacents à l'hétéroatome d'azote ne soient ni substitués, ni communs à deux cycles.

Conviennent plus particulièrement à la mise en oeuvre du présent procédé les hétérocycles azotés à 6 chaînons, de $pK_a$ compris entre 4 et 7, notamment la pyridine, la picoline-4, l'isoquinoléïne et la lutidine-3,5.

La quantité de base azotée tertiaire utilisée est en général telle que le rapport molaire N/Co soit compris entre 1 et 50. Pour une bonne mise en oeuvre de l'invention la Demanderesse préconise de fixer ce rapport à une valeur comprise entre 2 et 25.

L'une des caractéristiques essentielles du présent procédé réside dans l'utilisation à titre de solvant d'un hydrocarbure aromatique qui peut comporter, le cas échéant, de 1 à 3 substituants indépendamment choisis parmi les radicaux alkyles, aryles et aralkyles renfermant au maximum 20 atomes de carbone.

Plus spécifiquement, on fait appel à des solvants pouvant être représentés par la formule :

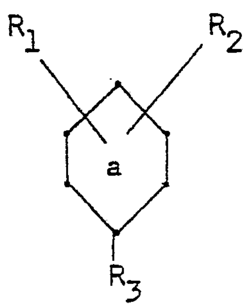

dans laquelle a est un noyau benzénique ou naphtalénique, $R_1$, $R_2$ et $R_3$, identiques ou différents, représentent l'hydrogène, un radical alkyle, aryle ou aralkyle qui renferme au maximum 20 atomes de carbone. De préférence, au moins un des radicaux $R_1$ à $R_3$ est un atome d'hydrogène, et les deux autres radicaux sont choisis parmi l'hydrogène, les radicaux alkyles ayant au maximum 10 atomes de carbone et le radical phényle.

De préférence, a est un noyau benzénique, deux des radicaux $R_1$ à $R_3$ représentent un atome d'hydrogène et le troisième radical est un

radical alkyle qui comporte au maximum 4 atomes de carbone ou un radical phényle.

Bien entendu on peut utiliser des mélanges de plusieurs de ces composés aromatiques et, notamment des mélanges couramment disponibles dans le commerce.

A titre d'exemples de solvants convenant à la mise en oeuvre du présent procédé on peut : le benzène, le naphtalène, le toluène, l'éthylbenzène, le cumène, le tertiobutylbenzène, le n-nonylbenzène, le n-octadécylbenzène, les méthylnaphtalènes, les isobutylnaphtalènes, le diphénylméthane, le diphényle, les xylènes, les diméthylnaphtalènes, le p-éthyltoluène, le p-heptyltoluène, les diéthylbenzènes, les méthylisopropylbenzènes (cymènes), les méthyldiphényles, le diméthyl-4,4' diphényle, les terphényles, le mésitylène et ses isomères, les éthylxylènes, les triméthylnaphtalènes .....

Le benzène et les monoalkylbenzènes dont le reste alkyle renferme au maximum 4 atomes de carbone, sont préconisés par la Demanderesse en raison d'une part des résultats satisfaisants auxquels ils conduisent et d'autre part, de leur plus grande disponibilité.

La quantité de solvant qui a une influence sur la sélectivité de la réaction sera en général supérieure à 20 % (en poids) du mélange réactionnel initial et, pour une bonne mise en oeuvre du présent procédé, elle sera comprise entre 30 et 60 % (en poids) dudit mélange.

Le procédé selon la présente invention est également conduit en présence d'hydrogène, l'hydrogène représentant au moins 0,1 % (en volume) du monoxyde de carbone.

Pour une bonne mise en oeuvre de l'invention l'hydrogène représentera au maximum 3 % (en volume) du monoxyde de carbone et, de préférence, il représentera de 0,5 à 2 % (en volume) du monoxyde de carbone.

Bien entendu, si l'hydrogène peut être introduit dans le milieu réactionnel, de manière commode, sous forme d'un mélange avec le monoxyde de carbone, il peut être aussi alimenté séparément.

La réaction est conduite en phase liquide à une température supérieure à 120 °C, sans qu'il soit utile de dépasser 200 °C, sous une pression de monoxyde de carbone d'au moins 50 bars et pouvant atteindre

1 000 bars. On préfère opérer à une température de l'ordre de 130 à 180 °C et sous une pression de monoxyde de carbone de l'ordre de 100 à 300 bars.

Le monoxyde de carbone utilisé peut renfermer, en plus de l'hydrogène, des impuretés telles que du dioxyde de carbone, du méthane et de l'azote.

En fin de réaction, ou lorsque la conversion souhaitée est atteinte on récupère l'adipate d'alkyle par tout moyen approprié, par exemple, par distillation ou extraction liquide-liquide.

Les exemples ci-après illustrent l'invention sans toutefois en limiter le domaine ou l'esprit.

- EXEMPLES -

Les conventions suivantes sont utilisées ci-après.

Dans les produits formés ne sont pas inclus les composés résultant de l'isomérisation de position de la double liaison oléfinique.

Les produits formés sont essentiellement les diesters et le pentanoate d'alkyle, ce dernier résultant de l'hydrogénation de l'ester de départ.

- A désigne l'activité exprimée en moles de produits formés par heure et par atome-gramme de cobalt.
- X (%) désigne le nombre de moles de diesters pour 100 moles de produits formés.
- Y (%) désigne le nombre de moles d'adipate d'alkyle pour 100 moles de produits formés.
- Z (%) désigne le nombre de moles de pentanoate d'alkyle pour 100 moles de produits formés.

- EXEMPLES 1 à 18 - Essais témoins (a) à (d) :

On a réalisé une série d'essais selon le mode opératoire suivant :

Dans un autoclave de 125 ml en acier inoxydable, purgé sous argon on introduit du pentène-3 oate de méthyle (P3), du méthanol, du dicobaltoctacarbonyle (DCOC), de l'isoquinoléïne, et le cas échéant un solvant.

L'autoclave est alors purgé par un courant de monoxyde de carbone renfermant, le cas échéant, de l'hydrogène. On porte ensuite

l'autoclave à la température (T), sous une pression (P). Après un temps de réaction (désigné par t et exprimé en heures), à cette température, l'autoclave est refroidi et dégazé. Le mélange réactionnel est analysé par chromatographie en phase gazeuse. Les conditions particulières ainsi que le résultats obtenus figurent respectivement dans les tableaux (A) et (B) ci-après :

Dans le tableau A les rapports MeOH/P3, P3/Co et N/Co désignent respectivement le rapport molaire du méthanol au pentène-3 oate (l'exemple 12 est réalisé au départ de pentène-2 oate de méthyle), le rapport du nombre de moles de pentène-3 oate au nombre d'atomes-grammes de cobalt, et le rapport du nombre de moles d'isoquinoléïne au nombre d'atomes-grammes de cobalt.

Les essais témoins (a) à (d) montrent clairement qu'en l'absence de solvant, la présence d'hydrogène se traduit par une augmentation de l'efficacité du catalyseur à base de cobalt et par une baisse notable de la sélectivité en adipate de diméthyle.

L'essai témoin (e) montre qu'en l'absence d'hydrogène, la présence de benzène dans le milieu réactionnel permet d'obtenir l'adipate de diméthyle avec une sélectivité remarquable. Toutefois l'efficacité du catalyseur à base de cobalt est très faible dans ces conditions. Les exemples 1 à 4 montrent que la présence simultanée de benzène et d'hydrogène permet d'obtenir sélectivement et efficacement l'adipate de diméthyle.

TABLEAU (A)

| Réf | P3 mmol | MeOH mmol | DCOC mmol | MeOH/ P3 | P3/Co | N/Co | $H_2$ (% vol) | P bars | T °C |
|---|---|---|---|---|---|---|---|---|---|
| a | 50,1 | 109 | 0,96 | 2,17 | 24,6 | 4 | 0 | 130 | 160 |
| b | 49,7 | 99 | 0,88 | 1,99 | 28,5 | 4,5 | 0,7 | " | " |
| c | 99,8 | 198 | 2,00 | 1,98 | 25,0 | 12 | 0,9 | " | " |
| d | " | 200 | 2,01 | 2,00 | 24,8 | " | 2,6 | " | " |
| e | 50,8 | 43 | 1,01 | 0,85 | 25,1 | 3,9 | 0 | " | " |
| 1 | 50,2 | 40 | 1,00 | 0,80 | " | 4,0 | 0,8 | " | " |
| 2 | 50,1 | 41 | 1,03 | 0,82 | 24,4 | 4,0 | 2 | " | " |
| 3 | 50,6 | 102 | 1,00 | 2,01 | 25,3 | 11,9 | 0,8 | " | " |
| 4 | 50,3 | " | 0,95 | 2,03 | 26,4 | 12,6 | 2 | " | " |
| f | 50,3 | 104 | 1,00 | 2,06 | 25,2 | 3,9 | 0,8 | " | " |
| 5 | 49,9 | 100 | 0,99 | 2,00 | 25,1 | 3,9 | " | " | " |
| 6 | 49,2 | 101 | 0,92 | 2,05 | 27,3 | 4,3 | " | " | " |
| 7 | 50,2 | 100 | 0,98 | 1,99 | 25,7 | 4,2 | " | " | " |
| 8 | 48,8 | 100 | 0,97 | 2,05 | 25,7 | 4,1 | " | " | " |
| g | 100 | 198 | 2,00 | 1,98 | 24,9 | 2,1 | " | " | " |
| 9 | 50,1 | 102 | 1,01 | 2,03 | 24,7 | 2,0 | " | " | " |
| h | 101 | 196 | 1,97 | 1,94 | 25,6 | 8,1 | " | " | " |
| 10 | 50,4 | 97 | 0,94 | 1,92 | 26,7 | 8,4 | " | " | " |
| i | 49,6 | 104 | 0,98 | 2,09 | 25,0 | 20,6 | " | " | " |
| 11 | 50,1 | 101 | 1,02 | 2,01 | 24,6 | 19,5 | " | " | " |
| 12 | 50,2 | 100 | 1,02 | 1,99 | 25 | 3,9 | 0,7 | " | " |
| 13 | 50,2 | 102 | 1,90 | 2,03 | 13,2 | 4,2 | 0,8 | " | " |
| 14 | 99,9 | 201 | 1,03 | 2,01 | 48,5 | 7,9 | " | 250 | 180 |
| 15 | 100 | 102 | 1,00 | 1,02 | 50,1 | 7,9 | " | " | " |
| j | 49,7 | 98,4 | 1,01 | 1,98 | 24,5 | 7,9 | " | 130 | " |
| 16 | 50,0 | 99,0 | 1,02 | " | 24,4 | 7,9 | " | " | " |
| 17 | 50,2 | 102 | 1,01 | 2,03 | 24,8 | 3,9 | 1 | " | 160 |
| 18 | 49,7 | 99 | 1,02 | 1,99 | 24,4 | 3,8 | " | " | " |

TABLEAU (B)

| Réf. | SOVLANT nature | (% pds) | t | A | X (%) | Y (%) | Z (%) |
|---|---|---|---|---|---|---|---|
| a | - | 0 | 1 | 3,7 | 95,1 | 79,4 | 4,9 |
| b | - | 0 | " | 10,4 | 89,0 | 74,6 | 10,4 |
| c | - | 0 | " | 1,9 | 94,7 | 75,5 | 4,9 |
| d | - | 0 | " | 3,3 | 92,5 | 74,7 | 6,8 |
| e | benzène | 51 | 2 | 1,2 | 97,3 | 83,5 | 2,7 |
| 1 | " | " | " | 4,6 | 97,0 | 83,7 | 2,1 |
| 2 | " | " | " | 5,3 | 92,4 | 79,6 | 6,7 |
| 3 | " | 41 | " | 5,2 | 94,7 | 80,0 | 5,0 |
| 4 | " | 42 | " | 5,0 | 93,8 | 79,3 | 5,6 |
| f | - | 0 | " | 7,9 | 92,3 | 76,4 | 7,0 |
| 5 | benzène | 20 | " | 6,9 | 92,5 | 77,6 | 6,4 |
| 6 | " | 46 | " | 4,5 | 95,2 | 82,4 | 4,2 |
| 7 | toluène | 46 | " | 2,9 | 95,3 | 81,9 | 4,1 |
| 8 | t-butylbenzène | 47 | " | 3,4 | 95,1 | 81,7 | 4,1 |
| g | - | 0 | " | 5,6 | 94,8 | 78,2 | 4,5 |
| 9 | benzène | 48 | " | 2,1 | 94,1 | 80,0 | 4,4 |
| h | - | 0 | " | 2,4 | 94,9 | 76,6 | 4,6 |
| 10 | benzène | 44 | " | 5,3 | 95,1 | 80,5 | 4,0 |
| i | - | 0 | " | 1,1 | 90,1 | 73,7 | 8,8 |
| 11 | benzène | 38 | " | 3,9 | 94,5 | 77,2 | 5,2 |
| 12 | " | 46 | " | 7,6 | 95,3 | 81,6 | 4,2 |
| 13 | " | 60 | " | 2,6 | 95,2 | 81,7 | 4,2 |
| 14 | " | 31 | " | 13,9 | 95,1 | 80,8 | 4,4 |
| 15 | " | 52 | " | 5,4 | 95,5 | 78,3 | 3,9 |
| j | - | 0 | " | 5,6 | 88,0 | 74,8 | 11,6 |
| 16 | benzène | 44 | " | 7,4 | 91,6 | 79,3 | 8,1 |
| 17 | naphtalène | 50 | " | 4,6 | 96,3 | 82,4 | 3,7 |
| 18 | biphényle | 50 | " | 5,4 | 95,0 | 81,3 | 4,3 |

## REVENDICATIONS

1. - Procédé de préparation d'adipates d'alkyle par réaction d'un alcool et du monoxyde de carbone sur des pentènoates d'alkyle en présence d'un catalyseur métallique, choisi dans le groupe constitué pa le cobalt et ses composés et d'une base azotée tertiaire, caractérisé e ce que la réaction est effectuée en présence d'hydrogène, l'hydrogène représentant au moins 0,1 % (en volume) du monoxyde de carbone, et dans un hydrocarbure aromatique qui peut comporter, le cas échéant, de 1 à 3 substituants indépendamment choisis parmi les radicaux alkyles, aryles aralkyles renfermant au maximum 20 atomes de carbone.

2. - Procédé selon la revendication 1, caractérisé en ce que l'hydrocarbure aromatique utilisé est choisi parmi les composés de formule :

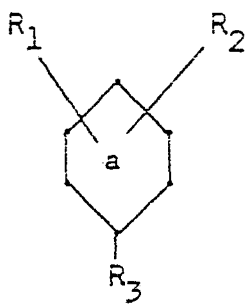

dans laquelle a est un noyau benzénique ou naphtalénique, $R_1$, $R_2$ et $R_3$, identiques ou différents, représentent l'hydrogène, un radical alkyle, aryle ou aralkyle ayant au maxium 20 atomes de carbone.

3. - Procédé selon la revendication 2, caractérisé en ce que l'un au moins des radicaux $R_1$ à $R_3$ est un atome d'hydrogène et les deux autres radicaux sont choisis parmi l'hydrogène, les radicaux alkyl ayant au maximum 10 atomes de carbone et le radical phényle.

4. - Procédé selon la revendication 2 ou 3, caractérisé en ce que a est un noyau benzénique.

5. - Procédé selon l'une quelconque des revendications précédentes, caractérisé en ce que l'hydrocarbure aromatique utilisé comme solvant est choisi parmi le benzène et les monoalkylbenzènes dont le reste alkyle renferme au maximum 4 atomes de carbone.

6. - Procédé selon l'une quelconque des revendications précédentes, caractérisé en ce que l'hydrocarbure aromatique représente au moins 20 % en poids du mélange réactionnel initial.

BAD ORIGINAL

7. - Procédé selon la revendication 6, caractérisé en ce que l'hydrocarbure aromatique représente de 30 à 60 % en poids du mélange réactionnel initial.

8. - Procédé selon l'une quelconque des revendications précédentes, caractérisé en ce que l'hydrogène représente au maximum 3 % (en volume) du monoxyde de carbone.

9. - Procédé selon l'une quelconque des revendications précédentes, caractérisé en ce que le rapport atomique N/Co est compris entre 1 et 50 et, de préférence entre 2 et 25.

10. - Procédé selon l'une quelconque des revendications précédentes, caractérisé en ce que la température de réaction est comprise entre 120 et 200 °C, de préférence, entre 130 et 180 °C.

11. - Procédé selon l'une quelconque des revendications précédentes, caractérisé en ce que la pression est comprise entre 50 et 1 000 bars et, de préférence, entre 100 et 300 bars.

Office européen des brevets

# RAPPORT DE RECHERCHE EUROPEENNE

Numéro de la demande

EP 83 42 0062

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | US-A-3 996 164 (AKIO MATSUDA) * Colonne 2, lignes 26-61; colonne 13, exemple 6 * --- | 1 | C 07 C 69/44<br>C 07 C 67/38 |
| A | FR-A-2 384 738 (BASF) * Page 1, lignes 1-10; page 7, lignes 6-26 * --- | | |
| D,X | BULLETIN OF THE CHEMICAL SOCIETY OF JAPAN, vol. 46, no. 2, fevrier 1973, pages 524-530, Tokio, JP. A. MATSUDA: "The cobalt carbonyl-catalyzed hydroesterification of butadiene with carbon monoxide and methanol" * Page 526, dernier alinéa, colonne de droite; page 527, colonne de gauche, tableau 6 * ----- | 1-6,9-11 | |
| | | | DOMAINES TECHNIQUES RECHERCHES (Int. Cl. 3) |
| | | | C 07 C 69/00<br>C 07 C 67/00 |

Le présent rapport de recherche a été établi pour toutes les revendications

| Lieu de la recherche | Date d'achèvement de la recherche | Examinateur |
|---|---|---|
| LA HAYE | 23-06-1983 | KINZINGER J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82